# EUROPÄISCHE PATENTANMELDUNG

(11) **EP 1 321 101 A2**
(43) Veröffentlichungstag der Anmeldung: **25.06.2003**
(21) Anmeldenummer: 02102789.1
(22) Anmeldetag: 18.12.2002
(51) Int. Cl.: A61B 6/12, A61B 6/00

(54) **Verfahren zur Unterstützung der Orientierung im Gefässsystem**

(30) Priorität: 19.12.2001 DE 10162272
(71) Anmelder: Philips Intellectual Property & Standards GmbH, 20099 Hamburg (DE); Koninklijke Philips Electronics N.V., 5621 BA Eindhoven (NL)
(72) Erfinder: Mollus, Sabine, 52088, Aachen (DE); Eck, Kai, 52088, Aachen (DE)
(74) Vertreter: Volmer, Georg, Dipl.-Ing.

(57) **Zusammenfassung**

Die Erfindung betrifft ein Verfahren, mit welchem bei einer invasiven Gefäßoperation eine Gefäßkarte mit großer Genauigkeit bereitgestellt und der aktuellen Röntgenaufnahme zugeordnet werden kann. Die Gefäßkarte wird dabei aus einem vierdimensionalen Modell des Gefäßbaums gewonnen, das heißt aus einem räumlich-dreidimensionalen, zeitabhängigen Modell, wobei sich die Zeitabhängigkeit auf Körpereigenbewegungen wie insbesondere den Herzschlag und/oder die Atmung bezieht. Insbesondere kann der Rhythmus des Herzschlages oder der Atmung sensorisch erfasst und zur synchronen Auswahl und Darstellung des entsprechenden dreidimensionalen Gefäßmodells verwendet werden.

## Beschreibung

Die Erfindung betrifft ein Verfahren zur Unterstützung der Orientierung im Gefäßsystem eines Körpervolumens. Ferner betrifft sie ein Röntgengerät zur Überwachung einer invasiven Gefäßoperation.

Aus der DE 100 47 314 A1 ist ein Verfahren zur Unterstützung der Orientierung während einer Angioskopie bekannt, bei welchem ein dreidimensionales Modell des Gefäßbaumes der Blutgefäße verwendet wird. Hierbei wird in einem Röntgenfluoroskop während des angioskopischen Eingriffs die Position eines Katheters oder eines Führungsdrahtes in einer zweidimensionalen Projektion verfolgt, wobei der Arzt die Projektionsrichtung nach Wunsch verändern kann. Die aktuelle Projektionsrichtung wird jeweils erfasst, und es wird die entsprechende Projektion des dreidimensionalen Modells des Gefäßbaumes berechnet. Letztere kann dann dem aktuellen röntgenfluoroskopischen Bild überlagert werden, wobei insbesondere in einer "virtuellen Kontrastmittelinjektion" die aus dem Modell bestimmten Gefäßverläufe im Bereich der Katheterspitze im fluoroskopischen Livebild hervorgehoben dargestellt werden können. Nachteilig bei diesem Verfahren ist jedoch, dass es insbesondere in kleinen Gefäßen häufig zu einer unzureichenden räumlichen Übereinstimmung zwischen dem röntgenfluoroskopischen Livebild und der Projektion des Modells kommt.

Vor diesem Hintergrund war es eine Aufgabe der vorliegenden Erfindung, ein verbessertes Verfahren und ein verbessertes Röntgengerät bereitzustellen, welche eine höhere Präzision bei der Kombination eines Durchleuchtungsbildes mit einem Gefäßmodell bieten.

Diese Aufgabe wind durch ein Verfahren mit den Merkmalen des Anspruchs 1 sowie durch ein Röntgengerät mit den Merkmalen des Anspruchs 9 gelöst. Vorteilhafte Ausgestaltungen sind in den Unteransprüchen enthalten.

Das erfindungsgemäße Verfahren dient der Unterstützung der Orientierung im Gefäßsystem eines Körpervolumens Es kann insbesondere bei invasiven Gefäßoperationen nutzbringend eingesetzt werden, welche sowohl diagnostischen als auch therapeutischen Zwecken dienen können und in der Regel mit Hilfe eines Katheters beziehungsweise eines Führungsdrahtes durchgeführt werden. Das Verfahren enthält die folgenden Schritte:
a) Die Gewinnung von Modellen des Gefäßsystems in dem betrachteten Körpervolumen zu verschiedenen Phasen einer Eigenbewegung des Körpers. Unter einem "Modell" ist in diesem Sinne jede zur geometrischen Beschreibung des Gefäßsystems geeignete Menge von Daten zu verstehen, wobei es sich insbesondere um eine binäre oder vektorielle Darstellung des Gefäßbaumes handeln kann. Vorzugsweise sind die Modelle dreidimensional, so dass sie die räumliche Form des Gefäßbaumes repräsentieren. Bei der Eigenbewegung des Körpers kann es sich insbesondere um eine zyklische Eigenbewegung wie den Herzschlag und/oder die Atmung handeln.
b) Die Erzeugung einer grafischen Darstellung basierend auf mindestens einem der Modelle des Gefäßsystems und einer aktuellen Aufnahme des Körpervolumens. Bei der Aufnahme kann es sich insbesondere um eine Durchleuchtungsaufnahme wie etwa eine Röntgenaufnahme handeln, welche im Folgenden häufig stellvertretend erwähnt und betrachtet wird. Grundsätzlich ist das Verfahren hierauf jedoch nicht beschränkt und kann z.B. auch bei Kemspinresonanz-Verfahren (MNR), sonografische Verfahren, szintigrafischen Verfahren oder dergleichen angewendet werden

Bei dem erfindungsgemäßen Verfahren findet eine Berücksichtigung von Eigenbewegungen des Körpers wie insbesondere des Herzschlags und/oder der Atmung statt, welche einen erheblichen Einfluss auf die Form und Lage der Gefäße haben. So verändert beispielsweise der Herzschlag die Form des Herzens und seiner Gefäße und mittelbar auch die Lage von Gefäßen in benachbarten Organen. Ähnliches gilt für die Lunge beziehungsweise den Brustraum bei der Atmung Erfindungsgemäß werden solche Eigenbewegungen des Körpers dadurch berücksichtigt, dass für verschiedenen Phasen dieser Eigenbewegungen verschiedene Modelle des Gefäßsystems gewonnen und verwendet werden. Auf diese Weise wird eine präzise Orientierung im betrachteten Körpervolumen möglich, wodurch nicht zuletzt Belastungen des Patienten durch Kontrastmittel und Röntgenstrahlung reduziert werden können.

Gemäß einer bevorzugten Weiterbildung des Verfahrens wird die aktuelle Eigenbewegung des Körpers über geeignete Sensoren erfasst, und es wird in Schritt b) des Verfahrens das zur aktuellen Phase der Eigenbewegung am besten passende Modell des Gefäßsystems verwendet. Auf diese Weise wird automatisch sichergestellt, dass das verwendete Modell mit der Eigenbewegung des Körpers synchronisiert ist. Insbesondere kann in Schritt b) das zur jeweiligen Phase der Eigenbewegung des Körpers am besten passende Modell (dreidimensional oder zweidimensional) synchron auf einem Monitor dargestellt werden, wobei vorzugsweise auf einem zweiten Monitor parallel die aktuelle Aufnahme gezeigt wird.

Vorzugsweise wird in Schritt b) eine zweidimensionale Projektion eines dreidimensionalen Modells des Gefäßsystems verwendet. Eine solche zweidimensionale Projektion entspricht vom Darstellungstyp her einem üblicherweise zweidimensionalen Durchleuchtungsbild, so dass der Arzt beide Darstellungen gut miteinander vergleichen kann.

Die Richtung und/oder Ebene der zweidimensionalen Projektion eines dreidimensionalen Modells kann bei dem vorstehend erläuterten Vorgehen im einfachsten Falle vom Arzt unter Gesichtspunkten der Zweckmäßigkeit ausgewählt werden. Vorzugsweise wird jedoch die Projektionsrichtung und/oder -ebene der dreidimensionalen Modelle speziell so gewählt, dass sie der Projektionsrichtung und/oder -ebene des aktuellen Durchleuchtungsbildes entspricht. Bei einer parallelen Darstellung der Modellprojektion und des aktuellen Durchleuchtungsbildes entsprechen sich somit die Projektionen, so dass ein unmittelbarer Vergleich möglich ist.

Die Projektionsrichtung und/oder -ebene des aktuellen Durchleuchtungsbildes kann aus Daten gewonnen werden, die vom Aufnahmegerät übermittelt werden. Aufgrund mechanischer Toleranzen haben diese Daten jedoch in der Regel nur eine begrenzte Genauigkeit, so dass es typischerweise zu einem Winkelfehler von etwa ±2° kommt. Eine größere Übereinstimmung zwischen den Projektionsrichtungen und/oder -ebenen von aktuellem Durchleuchtungsbild und Modell kann erfindungsgemäß dadurch erzielt werden, dass das aktuelle Durchleuchtungsbild mit (Durchleuchtungs-) Aufnahmen des zugrundeliegenden Körpervolumens verglichen wird, welche zur Gewinnung der Modelle des Gefäßsystems erzeugt und verwendet wurden. Zur Gewinnung der Modelle des Gefäßsystems wird nämlich in der Regel das interessierende Körpervolumen aus verschiedenen Richtungen, vorzugsweise in einer Rotationsbewegung aus allen Richtungen aufgenommen, und anschließend wird aus den so erhaltenen vielen zweidimensionalen Projektionen der (dreidimensionale) Gefäßverlauf rekonstruiert. Bei dem vorgeschlagenen Vorgehen werden nun diejenigen der erhaltenen zweidimensionalen Projektionen, deren Projektionsrichtungen und/oder -ebenen nahe bei der vorläufig ermittelten Projektionsrichtung und/oder -ebene des aktuellen Durchleuchtungsbildes liegen, mit dem aktuellen Durchleuchtungsbild verglichen. Diejenige dieser Projektionen, welche die größte Übereinstimmung mit dem aktuellen Durchleuchtungsbild zeigt, wird dann als eine Aufnahme aus derselben Richtung bzw. mit derselben Projektionsebene wie das Livebild angesehen, und die dieser Projektion zugrundeliegenden Parameter werden für die Auswahl der Projektionsrichtung und/oder -ebene des Modells verwendet.

Gemäß einer anderen Weiterbildung des Verfahrens wird die Position eines Objektes wie insbesondere eines Instrumentes, eines Katheters oder eines Führungsdrahtes aus der aktuellen Aufnahme ermittelt, und die korrespondierende Position in den Modellen des Gefäßsystems wird hervorgehoben dargestellt. Der Arzt kann somit unmittelbar in der Darstellung eines Modells des Gefäßsystems erkennen, wo sich das betreffende Objekt aktuell befindet. Eine weitere Möglichkeit zur Erzeugung einer grafischen Darstellung in Schritt b) des Verfahrens besteht darin, dass das mindestens eine Modell des Gefäßsystems der aktuellen Aufnahme überlagert wird. Durch die gemeinsame Darstellung in einem einzigen Bild lassen sich die Modelldaten unmittelbar zu den aktuellen Aufnahmedaten in Beziehung setzen.

Die Erfindung betrifft ferner ein Röntgengerät zur Überwachung einer invasiven Gefäßoperation, welches die folgenden Elemente enthält:
- Eine Röntgenstrahlungsquelle und einen Detektor zur Erzeugung von Röntgenaufnahmen eines vorgegebenen Körpervolumens Diese sind vorzugsweise drehbeweglich mit einer festen relativen Geometrie zueinander angeordnet. Typischerweise werden die Röntgenstrahlungsquelle und der Detektor an den Endpunkten eines sogenannten C-Armes befestigt, welcher um seinen Mittelpunkt drehbeweglich ist. Auf diese Weise kann die Aufnahmerichtung um den Körper eines Patienten herum auf einer Kreisbahn bewegt werden, so dass von einem im Mittelpunkt der Drehbewegung stehenden interessierenden Körpervolumen Aufnahmen aus verschiedenen Blickrichtungen gemacht werden können.
- Einen Speicher zur Speicherung von Modellen des Gefäßsystems des genannten Körpervolumens zu verschiedenen Phasen einer Eigenbewegung des Körpers. Die Modelle können dabei zuvor in einer separaten Untersuchung mit größerem Zeitabstand zur aktuellen Untersuchung oder auch während der aktuellen Untersuchung gewonnen worden sein. Sie können mit demselben Röntgengerät oder auch mit anderen Geräten wie etwa einem Röntgen-Computertomographen (CT) oder einem MNR Gerät gewonnen worden sein. Ferner können sie zweidimensional oder dreidimensional sein.
- Einen Sensor für die Körpereigenbewegung. Bei der Körpereigenbewegung kann es sich insbesondere um den Herzschlag und/oder die Atmung eines Patienten handeln. Der Sensor kann in diesem Falle ein EKG-Gerät oder ein zur Überwachung der Atmung geeignetes Gerät sein.
- Eine mit dem genannten Sensor und dem genannten Speicher gekoppelte Datenverarbeitungseinheit, welche so eingerichtet ist, dass sie eine grafische Darstellung basierend auf mindestens einem der genannten Modelle und einer aktuellen Röntgenaufnahme des Körpervolumens erzeugt.

Das beschriebene Röntgengerät erlaubt eine sehr präzise Überwachung einer invasiven Gefäßoperation, da es in seinem Speicher ein zeitabhängiges Modell des Gefäßsystems bereithält und dieses für eine Kombination mit dem aktuellen Livebild aus dem Körper verwendet. Über den Sensor der Vorrichtung kann dabei der aktuelle Verlauf der Körpereigenbewegung verfolgt werden, so dass das jeweils hierzu passende Modell ausgewählt und synchron für die Erzeugung der grafischen Darstellung verwendet werden kann.

Gemäß einer Weiterbildung des Röntgengerätes enthält dieses eine Einrichtung zur Bestimmung der aktuellen Bildebene beziehungsweise Projektionsrichtung der Röntgenaufnahme. Eine derartige Einrichtung kann zum Beispiel die Winkelstellung der Röntgenstrahlungsquelle und/oder des Detektors messen und die so gewonnenen Daten an die Datenverarbeitungseinheit weiterleiten, damit sie in die Erzeugung der gemeinsamen Darstellung eingehen können.

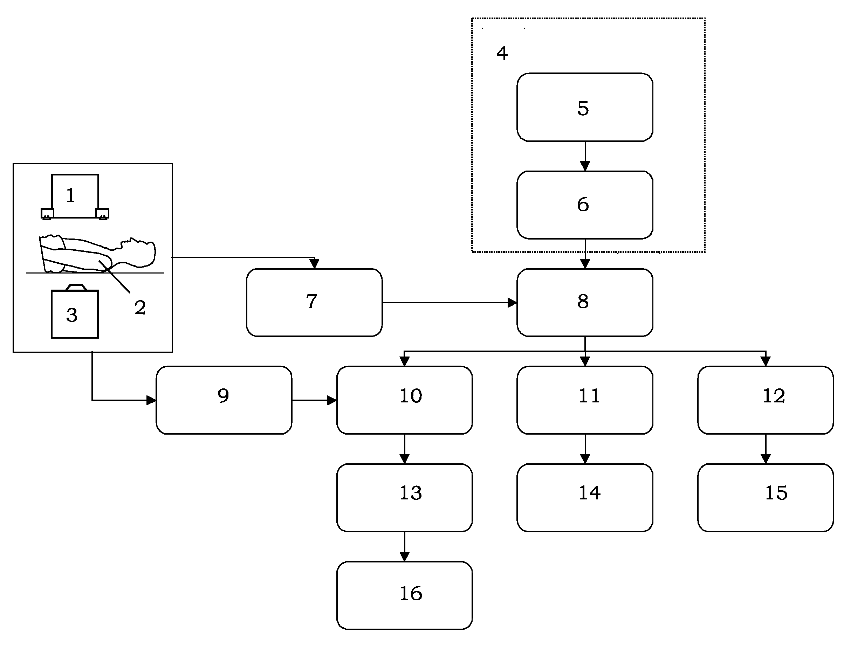

Im Folgenden wird die Erfindung mit Hilfe der Figur beispielhaft erläutert. Die einzige Abbildung zeigt schematisch die Komponenten eines erfindungsgemäßen Röntgengerätes.

Im linken Teil der Figur ist schematisch der bildgebende Teil eines Röntgengerätes bestehend aus einer Röntgenstrahlungsquelle 3 und einem Röntgendetektor 1 dargestellt, die typischerweise an einem drehbaren C-Arm angeordnet sind Zwischen den genannten Elementen liegt ein Patient 2, so dass ein interessierendes Körpervolumen dieses Patienten röntgenfluoroskopisch abgebildet werden kann. Vorliegend interessiert dabei insbesondere die Überwachung eines invasiven diagnostischen oder therapeutischen Eingriffs in der Gefäßbahn des Patienten. Dabei wird entlang der Gefäße ein Katheter (gegebenenfalls mit einem Führungsdraht) bis in oder bis vor das zu behandelnde Körpervolumen vorgeschoben. Die Navigation dieses Katheters wird üblicherweise von Serien-Röntgenaufnahmen unterstützt, welche dem Arzt die aktuelle Position des Katheters anzeigen. Hierbei werden in der Praxis der sogenannte "fluoroskopische Modus" geringerer Röntgendosis sowie der sogenannte "cine-Modus" höherer Röntgendosis unterschieden. Während des cine-Modus wird in der Regel Kontrastmittel über den Katheter in das Gefäßsystem injiziert, so dass die Topologie des Gefäßbaumes für eine Sequenz von vier bis fünf Herzzyklen sichtbar ist. Die Fluoroskopie wird in der Regel eingesetzt, um mit dem Führungsdraht (Guidewire) zu einer Verengung des Gefäßsystems ohne Kontrastmittel vorzustoßen und um das Instrument auf dem über die Verengung hinausgeschobenen Führungsdraht an der Verengung zu positionieren. Die richtige Lage wird nach der Positionierung mit einem kleinen Stoß Kontrastmittel unter Fluoroskopie verifiziert und gegebenenfalls in einem komplizierten iterativen Prozess korrigiert, der etliches an Zeit und Dosis in Anspruch nimmt. Wenn während des fluoroskopischen Modus kein Kontrastmittel eingesetzt wird, zeigen die Bildfolgen in diesem Modus nur den Katheter, den Führungsdraht und die Instrumente sowie einige Hintergrundinformationen an. Die Gefäße selbst sind aufgrund ihrer geringen Unterschiede in der Absorptionsdichte im Vergleich zum umliegenden Gewebe so gut wie nicht zu erkennen.

Der Arzt kann während des Eingriffes den Betrachtungswinkel und die Position des bildgebenden Systems verändern, um den Gefäßbaum, in welchem sich die Geräte gerade befinden, unter verschiedenen Perspektiven zu sehen. Zur Unterstützung des Arztes bei der Navigation im Gefäßsystem werden beim Stand der Technik nur statische Bilder ("Gefäßkarten" oder "roadmaps") verwendet, die aus mehreren vorangehenden Aufnahmen im cine-Modus ausgewählt werden. Derartige statische Abbildungen berücksichtigen jedoch nicht eine Eigenbewegung des Körpers, die insbesondere durch den Herzschlag und/oder die Atmung des Patienten 2 erzeugt wird
Zur Verbesserung dieser Situation wird erfindungsgemäß die Verwendung von zeitabhängigen, räumlich-dreidimensionalen Gefäßmodellen vorgeschlagen, die bei Definition der Zeit als vierte Dimension auch als "vierdimensionale" Gefäßmodelle bezeichnet werden können. Diese Modelle werden entweder in einem separaten Prozess 4 (lange) vor dem eigentlichen Eingriff gewonnen, oder sie können während des Eingriffes selbst erzeugt werden. Die Erzeugung der Modelle kann insbesondere durch eine sogenannte "rotierende Angiografie" erfolgen, bei welcher die Röntgenstrahlungsquelle 3 und der Detektor 1 um den Patienten 2 herum rotieren und währenddessen Aufnahmen aus verschiedenen Richtungen erzeugen. Der Speicher der Modelle ist in der Figur in Block 5 dargestellt.

In einem weiteren, durch den Block 6 symbolisierten Vorverarbeitungsschritt werden vorbereitend verschiedene Projektionen der Modelle aus Block 5 berechnet. Dabei handelt es sich um Projektionen, die durch zwei Winkelwerte entsprechend der geometrischen Blickrichtung sowie durch zwei Phasenwinkel (gemessen zwischen 0 und 2π) des Herzschlag-Zyklus und des Atmungszyklus definiert werden. Aufgrund des hohen Rechenaufwandes für die Erzeugung dieser Projektionen werden diese vorzugsweise vorab berechnet und nicht in Echtzeit ausgeführt. Es ist jedoch auch eine Berechnung in Echtzeit möglich, wenn eine entsprechende Rechenleistung zur Verfügung gestellt wird und/oder wenn der Aufwand z.B. durch Verwendung einer geringeren Auflösungsstufe reduziert wird.

Während des eigentlichen Untersuchungsverfahrens werden die Verarbeitungsschritte 7 bis 16 ausgeführt. Dabei werden zunächst vom Arzt röntgenfluoroskopische Bildfolgen erzeugt (Block 9). Um verschiedene Perspektiven des Gefäßbaumes betrachten zu können, ändert der Arzt die Winkel und die Position des bildgebenden Systems 1, 3 nach Bedarf. Die aktuellen Positionsdaten des Systems werden ständig von einem Positionserfassungssystem 7 bestimmt.

In Block 8 kann eine Auswahl derjenigen Projektion aus den vorberechneten Projektionen 6 erfolgen, deren Parameter mit den aktuellen, vom Positionserfassungssystem 7 bereitgestellten Daten am besten übereinstimmen. Da das bildgebende System 1, 3 sechs Freiheitsgrade für seine Positionierung aufweist, kann die in Block 8 ausgewählte Projektion des Gefäßsystems oder "roadmap" auch als "6F-roadmap" bezeichnet werden.

Für die Korrelation und Kombination der aktuellen fluoroskopischen Bildsequenz 9 und der aus den dreidimensionalen Gefäßmodellen bestimmten 6F-roadmaps gibt es verschiedene Möglichkeiten, von denen drei in der Figur dargestellt sind

Im einfachsten Fall wählt der Benutzer in Block 11 unter den bereitgestellten 6F-roadmaps oder Gefäßkarten eine von ihm bevorzugte aus. Hierbei handelt es sich typischerweise um eine solche, die zu einer spezifischen Phase des Herzzyklus oder Atemzyklus gehört. Diese Gefäßkarte wird dann auf einem zweiten Monitor oberhalb des Monitors, welcher das fluoroskopische Livebild zeigt, dargestellt (Block 14).

Gemäß einer zvwiten, in den Blöcken 12 und 15 dargestellten Variante wird mit zusätzlichen Sensoren (nicht dargestellt) das EKG und die Atmung des Patienten erfasst, und abhängig von den so ermittelten aktuellen Phasen von Herzschlag und Atmung wird automatisch eine dazugehörige Gefäßkarte aus Block 8 ausgewählt. Diese zum aktuellen Körperzustand synchrone Gefäßkarte wird dann in Block 15 auf einem zweiten Monitor parallel zum fluoroskopischen Livebild dargestellt.

In den Blöcken 10,13 und 16 ist schließlich eine weitere Darstellungsmöglichkeit veranschaulicht, bei dem in aufwendigeren Verfahren statisch oder dynamisch Indikatoren für die Instrumente integriert werden. Dabei wird zunächst in Block 10 eine Registrierung zwischen dem fluoroskopischen Livebild und dem dreidimensionalen Gefäßmodell beziehungsweise seiner zweidimensionalen Projektion (Gefäßkarte) durchgeführt, das heißt, dass einander entsprechende Punkte in beiden Bildern herausgesucht werden. In Block 13 wird dann eine Extraktion des Instrumentes wie etwa der Katheterspitze vorgenommen. Geeignete Algorithmen hierfür sind zum Beispiel in der WO 00/39753 beschrieben. Im Block 16 können dann statisch oder dynamisch Indikatoren für das Instrument in dem fluoroskopischen Bild und/oder in einer Darstellung des Gefäßmodells angebracht werden Ferner kann nach der Registrierung aus Block 10 auch eine digitale Subtraktionsangiografie (DSA) durchgeführt werden, bei welcher die Differenz aus dem aktuellen Bild und einem Referenzbild gebildet wird, um nicht interessierende Strukturen zu schwächen und interessierende Strukturen zu verstärken.

## Patentansprüche

1. Verfahren zur Unterstützung der Orientierung im Gefäßsystem eines Körpervolumens, enthaltend die Schritte:
a) Gewinnung von vorzugsweise dreidimensionalen Modellen des Gefäßsystems in dem Körpervolumen für verschiedene Phasen einer Eigenbewegung des Körpers;
b) Erzeugung einer grafischen Darstellung basierend auf mindestens einem der Modelle und auf einer aktuellen Aufnahme wie insbesondere einer Röntgenaufnahme des Körpervolumens.

2. Verfahren nach Anspruch 1,
wobei die Eigenbewegung des Körpers erfasst und in Schritt b) das der aktuellen Phase der Eigenbewegung am besten entsprechende Modell des Gefäßsystems verwendet wird

3. Verfahren nach Anspruch 1,
**dadurch gekennzeichnet,**
**dass** in Schritt b) eine zweidimensionale Projektion eines dreidimensionalen Modells dargestellt wird

4. Verfahren nach Anspruch 3,
**dadurch gekennzeichnet,**
**dass** die aktuelle Aufnahme eine Durchleuchtungsaufnahme ist und die Projektionsrichtung und/oder -ebene des dreidimensionalen Modells der Projektionsrichtung und/oder -ebene der Durchleuchtungsaufnahme entspricht.

5. Verfahren nach Anspruch 4,
**dadurch gekennzeichnet,**
**dass** die Projektionsrichtung und/oder -ebene der aktuellen Durchleuchtungsaufnahme aus einem Vergleich der Durchleuchtungsaufnahme mit Aufnahmen des Körpervolumens bestimmt wird, welche zur Gewinnung der dreidimensionalen Modelle des Gefäßsystems verwendet wurden.

6. Verfahren nach Anspruch 1,
**dadurch gekennzeichnet,**
**dass** die Position eines Objektes wie insbesondere eines Instrumentes, eines Katheters oder eines Führungsdrahtes aus der aktuellen Durchleuchtungsaufnahme ermittelt wird, und dass die hierzu korrespondierende Position in den Modellen des Gefäßsystems hervorgehoben dargestellt wird

7. Verfahren nach Anspruch 1,
**dadurch gekennzeichnet,**
**dass** in Schritt b) das mindestens eine Modell des Gefäßsystems einer Darstellung der Aufnahme überlagert wird

8. Verfahren nach Anspruch 1,
**dadurch gekennzeichnet,**
**dass** die Eigenbewegung des Körpers durch Herzschlag und/oder Atmung verursacht wird.

9. Röntgenaufnahmegerät zur Überwachung einer invasiven Gefäßoperation, enthaltend
- eine Röntgenstrahlungsquelle und einen Detektor zur Erzeugung von Röntgenaufnahmen eines vorgegebenen Körpervolumens;
- einen Speicher zur Speicherung von Modellen des Gefäßsystems in dem Körpervolumen zu verschiedenen Phasen einer Eigenbewegung des Körpers;
- einen Sensor für eine Eigenbewegung des Körpers;
- eine mit dem Sensor und dem Speicher gekoppelte Datenverarbeitungseinheit, welche so eingerichtet ist, dass sie eine grafische Darstellung basierend auf mindestens einem der Modelle des Gefäßsystems und einer aktuellen Röntgenaufnahme des Körpervolumens erzeugt.

10. Röntgenaufnahmegerät nach Anspruch 9,
**dadurch gekennzeichnet,**
**dass** es eine Einrichtung zur Bestimmung der aktuellen Projektionsrichtung und/oder -ebene der Röntgenaufnahme enthält.
